# EUROPÄISCHE PATENTSCHRIFT

(11) **EP 2 872 467 B1**
(45) Veröffentlichungstag und Bekanntmachung des Hinweises auf die Patenterteilung: **03.01.2018**
(21) Anmeldenummer: 13731294.8
(22) Anmeldetag: 19.06.2013
(51) Int. Cl.: C07C 1/24, C07C 45/50, C07C 209/26

(54) **ISONONYLAMINE AUSGEHEND VON 2-ETHYLHEXANOL, VERFAHREN ZU IHRER HERSTELLUNG**
ISONONYLAMINES FROM 2-ETHYLHEXANOL, PROCESSES FOR THEIR PREPARATION
ISONONYLAMINES PARTANT DE 2-ÉTHYLHEXANOL, PROCÉDÉ POUR LEUR PRÉPARATION

(30) Priorität: 13.07.2012 DE 102012014395
(43) Veröffentlichungstag der Anmeldung: 20.05.2015
(73) Patentinhaber: OXEA GmbH, 46147 Oberhausen (DE)
(72) Erfinder: THEUERKAUF, Jens, 47802 Krefeld (DE); FREY, Guido, D., 64560 Riedstadt (DE); EISENACHER, Matthias, 46485 Wesel (DE); KOCKRICK, Kristina, 40505 Düsseldorf (DE); STRUTZ, Heinz, 47445 Moers (DE)
(86) Internationale Anmeldenummer: PCT/EP2013/001812
(87) Internationale Veröffentlichungsnummer: WO 2014/008979

(56) Entgegenhaltungen:
- WO-A2-2009/146988
- DE-A1- 1 518 118
- US-A- 4 204 972
- US-A1- 2011 230 342

## Beschreibung

Die vorliegende Erfindung betrifft ein Verfahren zur Herstellung von Isononylaminen ausgehend von 2-Ethylhexanol, durch Dehydratisierung von 2-Ethylhexanol, Hydroformylierung des erhaltenen Octengemisches zu Isononanal und Umsetzung zu den entsprechenden Isononylaminen.

Aliphatische Amine sind wichtige organische Zwischenprodukte, die in großem industriellen Maßstab hergestellt werden. Beispielsweise werden sie für die Herstellung von pharmazeutischen Produkten, Agrochemikalien oder Farbstoffen weiterverarbeitet oder sie dienen als Zusatz in oberflächenaktiven Formulierungen, als Korrosionsinhibitor sowie als Additive in Schmiermitteln, beispielsweise in Form ihrer Dithiocarbamate oder entsprechender Salze, zur Verbesserung der Abriebsbeständigkeit mechanischer Apparaturen, die unter hohem Druck betrieben werden, oder als Hilfsstoffe für die Papier-, Textil- und Kautschukindustrie. Technische Bedeutung haben die kurzkettigen Alkylamine mit weniger als sechs Kohlenstoffatomen pro Alkylgruppe und die sogenannten Fettamine mit etwa acht bis 24 Kohlenstoffatomen pro Alkylkette. Während zunächst Fettamine aus nativen Fettsäuren hergestellt wurden, werden seit einigen Jahren Fettamine auch auf Basis petrochemischer Rohstoffe nach Verfahren gewonnen, die für die Herstellung kurzkettiger Amine schon seit vielen Jahren etabliert sind.

So führt die reduktive Aminierung von Aldehyden und Ketonen mit Ammoniak, primären oder sekundären Aminen zu primären, sekundären oder tertiären Aminen. Die Aminbildung kann beispielsweise durch folgende Reaktionsstufen beschrieben werden:

R¹-C(=O)-R² + R³NH₂ → R¹-C(=NR³)-R² + H₂O (1)

R¹-C(=NR³)-R² + H₂ → R¹-CHR²-NHR³ (2)

In der ersten Reaktionsstufe wird durch Umsetzung eines Aldehyds (R¹ gleich Alkyl und R² gleich Wasserstoff) oder eines Ketons (R¹ und R² gleich Alkyl) mit Ammoniak eine Iminzwischenstufe gebildet (R³ gleich Wasserstoff) oder mit einem primären Amin eine Azomethinzwischenstufe oder Schiffsche Base (R³ gleich Alkyl). Diese Zwischenstufen werden anschließend katalytisch hydriert, entweder direkt einstufig oder nach ihrer Isolierung wasserfrei in getrennten Reaktionsstufen. Die katalytische Hydrierung kann in Gegenwart gängiger Hydrierkatalysatoren, wie Nickel- oder Kobaltkatalysatoren, die mit Chromzusätzen aktiviert sind, erfolgen (DE 1257 782 A1, DE 2048 750 A1).

Werden sekundäre Amine mit Aldehyden oder Ketonen umgesetzt, so muss an dem zur Carbonylgruppe benachbarten Kohlenstoffatom ein Wasserstoffatom gebunden sein, das unter Ausbildung eines Enamins in Form von Wasser abgespalten werden kann. Die nachfolgende katalytische Hydrierung führt dann zu tertiären Aminen.

Setzt man Ammoniak mit Aldehyden oder Ketonen um, werden zunächst primäre Amine gebildet, die dann mit weiterem Aldehyd oder Keton über die Azomethinzwischenstufe zum sekundären Amin abreagieren, die dann noch analog zu tertiären Aminen weiterreagieren können. Die Produktverteilung kann durch den Ammoniakeinsatz gesteuert werden. Hohe molare Überschüsse an Ammoniak begünstigen die Bildung primärer Amine.

Neben der reduktiven Aminierung von Carbonylverbindungen wird auch die Aminierung von Alkoholen oder Ammonolyse in Gegenwart von Hydrierkatalysatoren technisch betrieben:

R¹-OH + HNR²R³ → R¹-NR²R³ + H₂O (3)

Wird Ammoniak umgesetzt (R² und R³ gleich Wasserstoff), bildet sich zunächst ein primäres Amin, das mit weiterem Alkohol zu einem sekundären Amin weiterreagiert, das analog zu einem tertiären Amin abreagieren kann. Auch bei dieser Reaktionsführung kann die Produktverteilung durch den Ammoniakeinsatz gesteuert werden. Ein hoher molarer Überschuss an Ammoniak fördert die Bildung des primären Amins.

Als Hydrierkatalysatoren eignen sich Nickel-, Kobalt-, Eisen- oder Kupferkatalysatoren, wie Raney Nickel (US 2782237, US 2182807). Die Aminierung von Alkoholen kann auch in Gegenwart von Wasserstoff erfolgen. US 2011/ 230342 A1 behandelt die Aminierung eines Isononanolgemisches mit Ammoniak in Gegenwart von Wasserstoff und einem Alkoholaminierungskatalysator zu Isononylaminen.

Weitere Verfahren zur Herstellung von Aminen umfassen die Umsetzung von Alkylhalogeniden mit Ammoniak, die Anlagerung von Ammoniak an olefinische Doppelbindungen, die katalytische Hydrierung von Carbonsäurenitrilen sowie die katalytische Reduktion von Nitroalkanen mit Wasserstoff (Ullmanns Encyklopädie der technischen Chemie, 4. Auflage, Band 7, 1974, Seiten 374-389; Band 11, 1976, Seiten 447-452).

Isononylamin (CAS-Nummer 27775-00-4) und Di-isononylamin (CAS-Nummer 28454-70-8) besitzen technische Bedeutung als Schmiermittelzusatz sowie als Additive in Schmiermitteln, beispielsweise in Form ihrer Dithiocarbamate oder entsprechender Salze, zur Verbesserung der Abriebsbeständigkeit mechanischer Apparaturen, die unter hohem Druck betrieben werden, als Zusatz in Korrosionsschutzmitteln oder für hydraulische Flüssigkeiten. Isononylamin enthält überwiegend das 3,5,5-Trimethylhexylamin und Di-isononylamin enthält überwiegend das Di-(3,5,5-Trimethylhexyl)amin als Hauptisomer.

Das C-9 Kohlenwasserstoffgerüst 3,5,5-Trimethylhexyl basiert auf dem petrochemischen Vorprodukt Isobuten, das in Gegenwart saurer Katalysatoren zu Diisobuten dimerisiert wird und von den dabei ebenfalls gebildeten höheren Oligomerisaten destillativ abgetrennt wird (Hydrocarbon Processing, April 1973, Seiten 171-173; Ullmann's Encyclopedia of Industrial Chemistry, 6th. Ed., 2003, Vol. 6, Seite 3). Diisobuten besteht im Wesentlichen aus den isomeren Octenen 2,4,4-Trimethyl-1-penten und 2,4,4-Trimethyl-2-penten und kann durch Oxo-Reaktion oder Hydroformylierungsreaktion mit Kohlenmonoxid und Wasserstoff in Gegenwart von Rhodium- oder Kobaltkatalysatoren in den entsprechenden Aldehyd 3,5,5-Trimethylhexanal umgewandelt werden (Ullmann's Encyclopedia of Industrial Chemistry, 6th. Ed., 2003, Vol. 2, Seite 68, 75; DE 2737633 A). Die Hydrierung liefert den Alkohol 3,5,5-Trimethylhexanol, den man beispielsweise als hochsiedendes Lösungsmittel verwendet (Ullmann's Encyclopedia of Industrial Chemistry, 6th. Ed., 2003, Vol.2, Seiten 22, 33).

Die bedeutendste Rohstoffquelle für Isobuten ist der C4-Schnitt aus der Dampfspaltung von Naphtha. Seine Verfügbarkeit im Vergleich zu den C2- und C3-Spaltprodukten kann durch die Bedingungen der Dampfspaltung gesteuert werden und richtet sich nach den Marktgegebenheiten.

Aus den C4-Spaltprodukten wird zunächst 1,3-Butadien durch Extraktion oder durch Selektivhydrierung in n-Butene entfernt. Das erhaltene C4-Raffinat, auch als Raffinat I bezeichnet, enthält überwiegend die ungesättigten Butene Isobuten, 1-Buten und 2-Buten sowie die hydrierten Produkte n-Butan und Isobutan. Aus dem Raffinat I wird im nächsten Schritt Isobuten entfernt und das erhaltene, isobutenfreie C4-Gemisch bezeichnet man als Raffinat II.

Für die Isobutenabtrennung werden in der industriellen Produktion verschiedene Verfahren angewandt, bei denen man die relativ höchste Reaktivität des Isobutens in dem Raffinat I ausnutzt. Bekannt ist die reversible protonenkatalysierte Wasseranlagerung zum tertiär-Butanol oder die Methanolanlagerung zum Methyl-tertiär-butylether. Aus diesen Additionsprodukten kann durch Rückspaltung wieder Isobuten zurückgewonnen werden (Weissermel, Arpe, Industrielle Organische Chemie, VCH Verlagsgesellschaft, 3. Auflage, 1988, S. 74-79).

Ebenfalls kann das butadienfreie C4-Raffinat bei erhöhter Temperatur und unter Druck mit einem sauren suspendierten Ionenaustauscher in Kontakt gebracht werden. Isobuten oligomerisiert zu Di-isobuten, Tri-isobuten und in geringem Maße zu höheren Oligomeren. Die Oligomeren werden von den nicht reagierten C4-Verbindungen abgetrennt. Aus dem Oligomerisat kann dann Di-isobuten oder Tri-isobuten destillativ rein gewonnen werden. Durch die Dimerisierung von n-Butenen mit Isobuten wird in geringem Maße Codimer gebildet (Weissermel, Arpe, Industrielle Organische Chemie, VCH Verlagsgesellschaft, 3. Auflage, 1988, Seite 77; Hydrocarbon Processing, April 1973, Seiten 171-173).

Di-isobuten, entweder hergestellt durch die Oligomerisierung von durch Rückspaltung erhaltenem reinen Isobuten oder gewonnen im Zuge der Aufarbeitung eines butadienfreien Raffinat I, wird anschließend über die Hydroformylierungsreaktion oder Oxo-Reaktion in ein um ein C-Atom verlängertes C9-Derivat überführt. Da Di-isobuten in überwiegendem Maße die Octene 2,4,4-Trimethyl-1-penten und 2,4,4-Trimethyl-2-penten enthält, ergibt die Hydroformylierungsreaktion den C9-Aldehyd 3,5,5-Trimethylhexanal als Hauptbestandteil. Weitere C9-Isomere, die in geringen Mengen zugegen sind, sind 3,4,4- und 3,4,5-Trimethylhexanal sowie 2,5,5-Trimethylhexanal, 4,5,5-Trimethylhexanal und 6,6-Dimethylheptanal.

Das so hergestellte Isononanal kann anschließend wie zuvor beschrieben über die reduktive Aminierung mit Ammoniak und Wasserstoff in Isononylamin oder Di-isononylamin umgewandelt werden. Auch kann Isononanal am Metallkontakt, beispielsweise an Nickel- oder Kobaltkatalysatoren, mit Wasserstoff zum Isononanol reduziert werden und anschließend über die Aminierungsreaktion in die entsprechenden Isononylamine derivatisiert werden. WO 2009/146988 A2 betrifft die Hydrierung eines Isononanalgemisches, hergestellt aus der kobaltkatalysierten Hydroformylierung von Dibuten, in einer zweistufen Verfahrensführung an sulfidfreien, metallhaltigen Katalysatoren.

Vor dem Hintergrund, dass die Verfügbarkeit an Octenen basierend auf dem C4-Schnitt aus der Naphthaspaltung beschränkt ist und von den lokalen Standortbedingungen abhängt, ist es wünschenswert, weitere Octenquellen auf Basis preiswert verfügbarer Großprodukte zu erschließen, die auf einfache Weise zu verschiedenen Standorten transportiert werden können. 2-Ethylhexanol steht als industrielles Großprodukt preiswert zur Verfügung, das ohne Probleme weitläufig vertrieben werden kann. 2-Ethylhexanol wird bekanntermaßen durch Hydroformylierung oder Oxo-Reaktion von Propylen zu n-Butyraldehyd mit nachfolgender alkalisch katalysierter Aldolkondensation zum 2-Ethylhexenal und anschließender Vollhydrierung zum 2-Ethylhexanol großtechnisch hergestellt (Ullmanns Encyklopädie der technischen Chemie, 4. Auflage, 1974, Verlag Chemie, Band 7, Seiten 214-215).

Auf die Verwendung von 2-Ethylhexanol zur Herstellung eines Octengemisches, das über die Dehydratisierung, Hydroformylierung und Hydrierung zu einem Isononanolgemisch verarbeitet wird, geht WO 03/029180 A1 kurz ein. Dabei steht die Einstellung der Viskosität der isomeren Phthalsäure-dialkylester im Mittelpunkt, die durch Veresterung von isomeren Nonanolen mit Phthalsäure oder Phthalsäureanhydrid erhalten werden. Hinweise, die Dehydratisierungsprodukte von 2-Ethylhexanol in Isononylamine zu überführen, werden nicht gegeben.

Die Nutzung von 2-Ethylhexanol als Octenquelle ermöglicht die Bereitstellung von Isononylaminen auf Basis von Propylen und mindert die Abhängigkeit von der Octenverfügbarkeit auf Butenbasis.

Die vorliegende Erfindung besteht daher in einem Verfahren zur Herstellung von Isononylaminen ausgehend von 2-Ethylhexanol. Das Verfahren ist dadurch gekennzeichnet, dass man
(a) 2-Ethylhexanol in Gegenwart eines Katalysators zu Octen dehydratisiert;
(b) das nach Schritt a) erhaltene Octen in Gegenwart einer Übergangsmetallverbindung der Gruppe VIII des Periodensystems der Elemente mit Kohlenmonoxid und Wasserstoff zu Isononanal umsetzt; und
(c) das nach Schritt b) erhaltene Isononanal in Isononylamine überführt.

Die Dehydratisierung von 2-Ethylhexanol kann sowohl in der Flüssigphase als auch in der Gasphase an einem dafür geeigneten Katalysator durchgeführt werden. Bevorzugt erfolgt die Dehydratisierung in der Gasphase bei Temperaturen im Bereich von 200 bis 450°C, vorzugsweise von 250 bis 380°C unter Verwendung fachüblicher Reaktoren in Gegenwart heterogener Katalysatoren mit dehydratisierenden Eigenschaften, wie Aluminiumoxid in seinen verschiedenen Modifikationen, Nickel niedergeschlagen auf Aluminiumoxid, oder Phosphorsäure niedergeschlagen auf Siliziumdioxid oder Aluminiumoxid. Solche zur Dehydratisierung geeignete Heterogenkatalysatoren sind aus dem Stand der Technik her bekannt (GB 313426, US 2468764, US 2919973) und stehen beispielsweise als Al3996 der Firma BASF SE kommerziell zur Verfügung. US 2919973 behandelt die Dehydratisierung von 2-Ethylhexanol an einem heterogenen Aluminiumoxidkatalysator bei Temperaturen um 350°C und bei einer Katalysatorbelastung von 2,4 bis 2,8 Liter 2-Ethylhexanol pro Liter Katalysator und Stunde. Der Stand der Technik gibt jedoch keine Auskunft über die Isomerenverteilung in dem erhaltenen Octengemisch.

Der in dem erfindungsgemäßen Verfahren für die Dehydratisierung von 2-Ethylhexanol eingesetzte Reaktor kann neben der Katalysatorschüttung noch weitere Füllkörper oder Einbauten enthalten, beispielsweise Raschigringe, Sättel, Pallringe, Filterplatten oder Kolonnenböden. Verwendet man Füllkörper, dann werden sie vorzugsweise oberhalb der Katalysatorschüttung angebracht, um das Totvolumen zu verringern. Wird in der Flüssigphase dehydratisiert, kann auf Einbauten und Füllkörper verzichtet werden, so dass in dem Reaktionsgefäß nur der Dehydratisierungskatalysator anwesend ist.

In der bevorzugten Arbeitsweise wird 2-Ethylhexanol in einem vorgeschalteten Verdampfer erhitzt und gasförmig über die Katalysatorschüttung geführt, gegebenenfalls unter Verwendung eines inerten Trägergases wie Stickstoff, Kohlendioxid oder Edelgase. Die Belastung V/Vh des heterogenen Katalysators kann über einen weiten Bereich variieren und beträgt im Allgemeinen von 0,2 bis 3,5 Liter 2-Ethylhexanol pro Liter Katalysator und Stunde. Das der Dehydratisierungszone entnommene Reaktionsgemisch wird anschließend kondensiert. Durch das abgespaltene Wasser fällt eine wässrige Phase an, die von der organischen Olefinphase durch einfache Phasentrennung separiert wird. Bei dem erhaltenen Octen handelt es sich um ein Gemisch strukturisomerer Octene mit den einfach verzweigten Octenen 2-Ethyl-1-hexen sowie cis/trans 3-Methyl-3-hepten und cis/trans 3-Methyl-2-hepten als Hauptkomponenten. Nennenswerte Mengen an Di-C8-Ethern werden nicht gebildet.

Das nach Entfernen des Spaltwassers vorliegende Octen wird anschließend ohne weitere Reinigung oder zweckmäßigerweise nach destillativer Aufreinigung für die Umsetzung mit Kohlenmonoxid und Wasserstoff in der Hydroformylierungsreaktion oder Oxo-Reaktion verwendet. Die eingesetzte Mischung aus Kohlenmonoxid und Wasserstoff bezeichnet man auch als Synthesegas. Man führt die Hydroformylierungsreaktion in einem homogenen Reaktionssystem durch. Der Begriff homogenes Reaktionssystem steht für eine im Wesentlichen aus Lösungsmittel, falls zugesetzt, Katalysator, olefinisch ungesättigter Verbindung und Reaktionsprodukt zusammengesetzte homogene Lösung. Als besonders wirksame Lösungsmittel haben sich die höher siedenden Kondensationsverbindungen der herzustellenden Aldehyde, insbesondere die Trimeren der herzustellenden Aldehyde, erwiesen, die als Nebenprodukte bei der Hydroformylierung anfallen, sowie ihre Mischungen mit dem herzustellenden Isononanal, so dass ein weiterer Lösungsmittelzusatz nicht unbedingt erforderlich ist. In einigen Fällen kann sich jedoch ein Lösungsmittelzusatz als zweckmäßig erweisen. Als Lösungsmittel werden organische Verbindungen eingesetzt, in denen Ausgangsmaterial, Reaktionsprodukt und Katalysator löslich sind. Beispiele für solche Verbindungen sind aromatische Kohlenwasserstoffe, wie Benzol und Toluol oder die isomeren Xylole und Mesitylen. Andere gebräuchliche Lösungsmittel sind Paraffinöl, Cyclohexan, n-Hexan, n-Heptan oder n-Octan, Ether, wie Tetrahydrofuran, Ketone oder Texanol® der Firma Eastman. Der Anteil des Lösungsmittels im Reaktionsmedium kann über einen weiten Bereich variiert werden und beträgt üblicherweise zwischen 20 und 90 Gew.-%, vorzugsweise 50 bis 80 Gew.-% bezogen auf das Reaktionsgemisch. Die Hydroformylierung des Octens kann aber auch ohne Lösungsmittelzusatz erfolgen.

Die Hydroformylierungsreaktion wird typischerweise in homogener organischer Phase in Gegenwart mindestens einer Übergangsmetallverbindung der Gruppe VIII des Periodensystems der Elemente durchgeführt. Die Umsetzung kann sowohl in Gegenwart sowie in Abwesenheit von komplexbildenden Organoelementverbindungen, die als Komplexliganden wirken, durchgeführt werden.

Wird die Hydroformylierungsreaktion in Gegenwart von Komplexliganden durchgeführt, eignet sich die Verwendung von Organophosphorverbindungen als Organoelementverbindungen. Derartige Komplexverbindungen und ihre Herstellung sind bekannt (US 3 527 809 A, US 4 148 830 A, US 4 247 486 A, US 4 283 562 A). Sie können als einheitliche Komplexverbindungen oder auch als Gemisch unterschiedlicher Komplexverbindungen eingesetzt werden. Die Übergangsmetallkonzentration im Reaktionsmedium erstreckt sich über einen breiten Bereich von etwa 1 bis etwa 1000 Gew.-ppm und beträgt vorzugsweise 10 bis 700 Gew.-ppm und insbesondere 25 bis 500 Gew.-ppm, jeweils bezogen auf das homogene Reaktionsgemisch. Als Katalysator kann die stöchiometrisch zusammengesetzte Übergangsmetall-Komplexverbindung Anwendung finden. Es hat sich jedoch als zweckmäßig erwiesen, die Hydroformylierung in Gegenwart eines Katalysatorsystems aus Übergangsmetall-Komplexverbindung und freiem Komplexliganden durchzuführen, der mit dem Übergangsmetall keine Komplexverbindung mehr eingeht. Der freie Komplexligand kann der Gleiche sein wie in der Übergangsmetall-Komplexverbindung, es können aber auch von diesem verschiedene Komplexliganden eingesetzt werden. Zu den bevorzugten Komplexliganden zählen Triarylphosphine wie Triphenylphosphin, Trialkylphosphine wie Tri(cyclohexyl) phosphin, Alkylphenylphosphine, organische Phosphite oder Diphosphite. Das molare Verhältnis von Übergangsmetall zu Komplexligand beträgt im Allgemeinen 1:1 bis 1:1000, es kann aber auch noch höher liegen. Bevorzugt setzt man das Übergangsmetall und den Komplexliganden in einem molaren Verhältnis von 1:3 bis 1:500 und insbesondere von 1:50 bis 1:300 ein.

Die Hydroformylierungsreaktion in Gegenwart von Komplexliganden bezeichnet man häufig auch als modifizierte Variante, die üblicherweise bei Temperaturen von 50 bis 180°C, vorzugsweise von 100 bis 160°C und Gesamtdrücken von 0,2 bis 30 MPa, vorzugsweise von 1 bis 20 MPa durchgeführt wird.

Die Hydroformylierungsreaktion kann ebenfalls in Abwesenheit von Komplexliganden nach der unmodifizierten Variante durchgeführt werden. Solche, beispielsweise nicht mit Phosphinen oder Phosphiten modifizierte Übergangsmetallkatalysatoren und ihre Eignung als Katalysator zur Hydroformylierung sind aus der Literatur her bekannt und sie werden als unmodifizierte Übergangsmetallkatalysatoren bezeichnet. Es wird in der Fachliteratur angenommen, dass die Übergangsmetallverbindung HM(CO)₄ die katalytisch aktive Übergangsmetallspezies bei der unmodifizierten Übergangsmetallkatalyse ist, obgleich dies aufgrund der vielen in der Reaktionszone nebeneinander ablaufenden Chemismen nicht eindeutig bewiesen ist. Vorzugsweise verwendet man als Übergangsmetalle der Gruppe VIII des Periodensystems der Elemente Kobalt, Rhodium, Iridium, Nickel, Palladium, Platin, Eisen oder Ruthenium und insbesondere Kobalt oder Rhodium. Der modifizierte oder unmodifizierte Übergangsmetallkatalysator bildet sich unter den Bedingungen der Hydroformylierungsreaktion aus den eingesetzten Übergangsmetallverbindungen, wie deren Salzen, wie Chloriden, Nitraten, Sulfaten, Acetaten, Pentanoaten, 2-Ethylhexanoaten oder Isononanoaten, deren Chalkogeniden, wie Oxiden oder Sulfiden, deren Carbonylverbindungen, wie M₂(CO)₈, M₄(CO)₁₂, M₆(CO)₁₆, M₂(CO)₉, M₃(CO)₁₂, deren Organoübergangsmetallverbindungen, wie Carbonylacetylacetonaten oder Cyclooctadienylacetaten oder -chloriden, in Gegenwart von Kohlenmonoxid/ Wasserstoffgemischen. Dabei kann die Übergangsmetallverbindung als Feststoff oder zweckmäßigerweise in Lösung eingesetzt werden. Als Übergangsmetallverbindung, die als Katalysatorvorstufe verwendet wird, eignet sich insbesondere Rhodiumisononanoat, Rhodiumacetat, Rhodium-2-ethylhexanoat oder Kobaltisononanoat, Kobaltacetat oder Kobalt-2-ethylhexanoat, oder Co₂(CO)₈, Co₄(CO)₁₂, Rh₂(CO)₈, Rh₄(CO)₁₂ oder Rh₆(CO)₁₆ oder Cyclopentadienylrhodiumverbindungen, Rhodiumacetylacetonat, oder Rhodiumdicarbonylacetylacetonat. Bevorzugt werden Rhodiumoxid und insbesondere Rhodiumacetat, Rhodium-2-ethylhexanoat und Rhodiumisononanoat eingesetzt.

Es ist aber auch möglich, den Übergangsmetallkatalysator in einer Vorcarbonylierungsstufe zunächst zu präformieren und ihn anschließend der eigentlichen Hydroformylierungsstufe zuzuführen. Die Bedingungen der Präformierung entsprechen dabei im Allgemeinen den Hydroformylierungsbedingungen.

Da im Allgemeinen die Verwendung von nicht mit Komplexliganden modifizierten Übergangsmetallkatalysatoren einen geringeren Übergangsmetallgehalt erfordert, arbeitet man bei der unmodifizierten Variante im Allgemeinen mit einer Übergangsmetallmenge von 1 bis 100 ppm, vorzugsweise 2 bis 30 ppm, bezogen auf das eingesetzt Octen. Ganz besonders wird Rhodium oder Kobalt in einer Menge von 2 bis 30 ppm, vorzugsweise von 5 bis 10 ppm, jeweils bezogen auf das eingesetzte Octen, verwendet.

Bei der Umsetzung des Octens mit Wasserstoff und Kohlenmonoxid zu Isononanal nach der unmodifizierten Variante arbeitet man zweckmäßigerweise bei höheren Drücken im Bereich von 5 bis 70 MPa, vorzugsweise von 5 bis 60 MPa und insbesondere von 10 bis 30 MPa. Geeignete Reaktionstemperaturen bewegen sich im Bereich von 50 bis 180°C, bevorzugt von 50 bis 150°C und insbesondere von 100 bis 150°C.

Die Zusammensetzung des Synthesegases, d. h. die Anteile von Kohlenmonoxid und Wasserstoff im Gasgemisch, kann in weiten Grenzen variiert werden. Im Allgemeinen setzt man Gemische ein, in denen das Molverhältnis von Kohlenmonoxid zu Wasserstoff 5 : 1 bis 1 : 5 beträgt. Üblicherweise ist dieses Verhältnis 1 : 1 oder weicht von diesem Wert nur wenig ab. Die olefinische Verbindung kann als solche oder in Lösung der Reaktionszone zugeführt werden. Geeignete Lösungsmittel sind Ketone wie Aceton, Methylethylketon, Acetophenon, niedrigere aliphatische Nitrile wie Acetonitril, Propionitril oder Benzonitril, Dimethylformamid, lineare oder verzweigte gesättigte aliphatische Monohydroxyverbindungen wie Methanol, Ethanol, Propanol und Isopropanol, aromatische Kohlenwasserstoffe wie Benzol oder Toluol und gesättigte cycloaliphatische Kohlenwasserstoffe wie Cyclopentan oder Cyclohexan.

Die Hydroformylierungsstufe kann sowohl diskontinuierlich als auch kontinuierlich durchgeführt werden. Die Gewinnung der gewünschten Aldehyde aus dem rohen Hydroformylierungsprodukt erfolgt nach konventionellen Verfahren, beispielsweise durch Destillation. Isononanal und weitere flüchtige Komponenten werden als Kopfprodukte abgezogen und, bei Bedarf, einer weiteren Feinreinigung unterzogen.

Die eingesetzten Übergangsmetallmengen fallen im Destillationsrückstand an und werden gegebenenfalls nach Zusatz von frischer Übergangsmetallverbindung und Entnahme eines Teils der im Verlauf der Reaktion gebildeten Aldehydkondensationsprodukte in die Reaktionszone zurückgeführt.

Das erhaltene Gemisch isomerer Isononanale wird aufgereinigt, zweckmäßigerweise durch Destillation, und anschließend über die reduktive Aminierung in Isononylamine umgewandelt. Unter reduktiver Aminierung im Sinne der vorliegenden Erfindung wird nicht nur die Reaktion des Isononanals mit Ammoniak, einem primären oder sekundären Amin mit Wasserstoff in Gegenwart eines gängigen Aminierungskatalysators verstanden, bei dem primäre, sekundäre und tertiäre Isononylamine gebildet werden, sondern auch die entsprechende Reaktion des Isononanols, obwohl bei der Alkoholaminierung oder Ammonolyse kein Wasserstoff verbraucht wird. Wird Isononanol als Ausgangsprodukt für die Aminsynthese eingesetzt, wird zunächst Isononanal in Gegenwart gängiger Hydrierkatalysatoren nach an sich bekannten Gasphasen- oder Flüssigphasenverfahren zum Isononanol hydriert. Als Hydrierkatalysatoren eignen sich beispielsweise Nickel- oder Kupferkatalysatoren, bevorzugt Nickelkatalysatoren. Die Hydrierungen erfolgen im Allgemeinen bei Wasserstoffdrücken von 6 bis 15 MPa und bei Temperaturen von 90 bis 150°C. In einer geeigneten Verfahrensführung wird in einer ersten Hydrierstufe am Kupferkatalysator in der Gasphase hydriert und nachfolgend in einer zweiten Hydrierstufe am Nickelkontakt in der Flüssigphase.

Die reduktive Aminierung sowohl des Isononanals als auch des Isononanols wird in fachüblichen Reaktoren, vorzugsweise an fest angeordneten Aminierungskatalysatoren durchgeführt. Geeignet sind beispielsweise Rohrreaktoren, unter denen man auch ein Bündel von mehreren eng parallel geschalteten Rohren versteht. Die eingesetzten Rohrreaktoren können ebenfalls Füllkörper oder Einbauten enthalten, beispielsweise Raschigringe, Sättel, Pallringe, Filterplatten oder Kolonnenböden, sowie gegebenenfalls Rührvorrichtungen. Die Suspensionshydrierung ist weniger geeignet. Sowohl die kontinuierliche Reaktionsführung als auch die absatzweise Verfahrensführung ist möglich.

Das Einsatzprodukt Isononanal oder Isononanol kann in Abhängigkeit vom gewünschten Aminierungsgrad im Über- oder Unterschuss an Ammoniak und Wasserstoff zur Reaktion gebracht werden. Im Allgemeinen werden je mol Einsatzprodukt wenigstens 0,2 mol, vorzugsweise von 0,3 bis 40 mol Ammoniak verwendet. Die Produktverteilung zwischen Isononylamin, Di-isononylamin und Tri-isononylamin kann durch den Ammoniakeinsatz gesteuert werden, wobei ein hoher Ammoniaküberschuss die Bildung des primären Isononylamins begünstigt. Neben Ammoniak können auch primäre oder sekundäre Amine wie Propylamin, n-Butylamin, 2-Ethylhexylamin, Di-n-propylamin, Di-n-butylamin oder Di-(2-ethylhexyl)amin eingesetzt werden, so dass gemischte Di-isononylamine und gemischte Tri-isononylamine erhalten werden. Die reduktive Aminierung kann lösungsmittelfrei ohne Zusatz eines Lösungs- oder Verdünnungsmittels oder unter Zusatz von Lösungsmitteln, wie beispielsweise Methanol oder Ethanol (DE 199 35 448 A1), durchgeführt werden.

Die der reduktiven Aminierung zugeführten Reaktionskomponenten können sich unter Reaktionsbedingungen in einem gasförmigen oder komprimierten Zustand befinden, wobei einzelne Reaktionskomponenten oder die Mischung selbst unterschiedliche Aggregatzustände einnehmen können. Im Allgemeinen wird die reduktive Aminierung bei Temperaturen im Bereich von 100 bis 200°C, vorzugsweise von 110 bis 150°C und bei Drücken im Bereich von 0,1 bis 40 MPa, vorzugsweise von 0,5 bis 30 MPa durchgeführt.

Für die reduktive Aminierung des Isononanals und des Isononanols verwendet man übliche Aminierungskatalysatoren, die mindestens ein Metall der Nebengruppe 8 bis 11 des Periodensystems der Elemente enthalten, wie Nickel, Kobalt, Platin, Palladium, Eisen, Rhodium oder Kupfer. Bevorzugt sind Nickel- oder Kobaltkatalysatoren. Neben trägerfreien Katalysatoren, wie Raney-Nickel oder Raney-Kobalt können auch geträgerte Katalysatoren eingesetzt werden. Als Katalysatorträger eignen sich alle herkömmlichen Trägermaterialien, zum Beispiel Aluminiumoxid, Aluminiumoxidhydrate in ihren verschiedenen Erscheinungsformen, Siliziumdioxid, Polykieselsäuren (Kieselgele) einschließlich Kieselgur, Kieselxerogele, Magnesiumoxid, Zinkoxid, Zirkoniumoxid und Aktivkohle. Neben den Hauptkomponenten katalytisch aktives Metall und Trägermaterial können die Aminierungskatalysatoren noch Zusatzstoffe in untergeordneten Mengen enthalten, die zum Beispiel der Verbesserung ihrer Aktivität und/oder ihrer Standzeit und/oder ihrer Selektivität dienen. Derartige Zusatzstoffe sind bekannt, zu ihnen gehören zum Beispiel die Oxide des Calciums, Bariums, Zinks, Aluminiums, Zirkoniums und Chroms. Als bevorzugtes katalytisch aktives Metall hat sich Nickel erwiesen. Insbesondere sind Nickelkatalysatoren auf Kieselgur als Trägermaterial und mit Chrom als Zusatzstoff für die reduktive Aminierung geeignet.

Das dem Reaktor entnommene Reaktionsgemisch wird über einen Hochdruckabscheider und nachfolgende Entspannungseinrichtungen auf Normaldruck entspannt und die erhaltenen rohen Isononylamine nach an sich bekannten Verfahren, beispielsweise durch Destillation, zu spezifikationsgerechter Ware aufgereinigt.

Bei den nach dem erfindungsgemäßen Verfahren hergestellten Isononylaminen ausgehend von 2-Ethylhexanol handelt es sich, je nach Aminierungsbedingungen, um das primäre Isononylamin, Di-isononylamin oder Tri-isononylamin sowie um gemischte sekundäre oder tertiäre Amine, die mindestens einen Isononylrest auf Basis von 2-Ethylhexanol enthalten. Für den Isononylrest steht ein C9-Kohlenwasserstoffrest, der im Wesentlichen in α-Position unverzweigt oder einfachverzweigt ist.

Der folgende Gegenstand (Verwendungen des erhaltenen Produkts), stellt keinen Teil der vorliegenden Erfindung dar. Die erhaltenen Isononylamine eignen sich besonders zur Verwendung als Korrosionsschutzmittel in Schmiermitteln.

Ebenfalls können die erhaltenen Isononylamine als Hilfsmittel in Gummiformulierungen sowie als Vulkanisationsbeschleuniger verwendet werden. Ebenfalls finden sie Verwendung als Additive in Schmiermitteln, beispielsweise in Form ihrer Dithiocarbamate oder entsprechender Salze, wie Molybdän-, Zink- oder Natriumdithiocarbamate, zur Verbesserung der Abriebsbeständigkeit mechanischer Apparaturen, die unter hohem Druck betrieben werden.

In den folgenden Beispielen wird die Herstellung von Isononylaminen ausgehend von 2-Ethylhexanol beschrieben.

### Beispiele

### I. Dehydratisierung von 2-Ethylhexanol

Zur Dehydratisierung wurde ein Quarzrohr mit einer Länge von 1,3 Meter und einem Durchmesser von 0,03 Meter verwendet, bei dem sich die beheizte Zone über 1,1 Meter erstreckte. Das Quarzrohr wurde mit 250 ml des sauren Katalysators Al 3996 der Firma BASF SE in Form von 3x3 Millimeter großen Tabletten bestückt. Das Totvolumen wurde mit Glasringen aufgefüllt. 2-Ethylhexanol wurde in einem vorgeschalteten Verdampfer verdampft und mit Hilfe eines Stickstoffstromes als Trägergas bei Normaldruck über die Katalysatorschüttung bei einer Temperatur von 350°C und mit einer Belastung von 0,5 Liter je Liter Katalysatorvolumen und Stunde gefahren. Das erhaltene Reaktionsgemisch wurde in einem nachgeschalteten Auffanggefäß kondensiert und die wässrige Phase wurde abgetrennt. Die angefallene organische Phase wies folgende gaschromatographisch ermittelte Zusammensetzung auf (FI.-%, gemäß DIN 51405):

| | |
|---|---|
| Vorlauf/C4-C7-Kohlenwasserstoffe | 0,3 |
| andere C8-Olefine | 9,6 |
| 2-Ethyl-1-hexen | 7,6 |
| cis-3-Methyl-3-hepten | 14,6 |
| trans-3-Methyl-3-hepten | 28,8 |
| cis-3-Methyl-2-hepten | 16,2 |
| trans-3-Methyl-2-hepten | 23,9 |
| n-Octene | 0,8 |
| Nachlauf | 0,1 |

### II. Hydroformylierung des gemäß Schritt I. erhaltenen Octens

Das nach Schritt I erhaltene rohe Octen wurde in Gegenwart von 5 ppm Rhodium, zugegeben in Form einer Lösung von Rhodium-2-ethylhexanoat in 2-Ethylhexanol und bezogen auf Octeneinsatz, bei einer Temperatur von 140°C und bei einem Synthesegasdruck von 19 MPa über einen Zeitraum von drei Stunden hydroformyliert. Die molare Zusammensetzung des Synthesegases betrug 1 Mol Wasserstoff zu 1 Mol Kohlenmonoxid. Das erhaltene rohe Hydroformylierungsprodukt wies folgende gaschromatographisch ermittelte Zusammensetzung (FI.-%, gemäß DIN 51405) auf:

| | |
|---|---|
| Vorlauf | 0,1 |
| C8-Kohlenwasserstoffe | 8,5 |
| Zwischenlauf | 0,2 |
| Isononanal | 88,1 |
| n-Nonanal | 1,4 |
| Nachlauf | 1,7 |

Die Ergebnisse weiterer Hydroformylierungsversuche mit einem über die Dehydratisierung von 2-Ethylhexanol erhaltenen Octen sind in der nachfolgenden Tabelle 1 zusammengestellt. Vor Einsatz wurde das rohe Octen an einer Claisenbrücke zur Nachlaufabtrennung bei einer Kopftemperatur von 119-122°C und bei Normaldruck destilliert. Die Einsatzoctene sowie die erhaltenen Reaktionsprodukte wurden gaschromatographisch analysiert (Angabe in FI.-%, gemäß DIN 51405).

**Tabelle 1: Hydroformylierung von Octenen, erhalten durch die 2-Ethylhexanoldehydratisierung**

| **Beispiel** | **IIa** | **IIb** |
|---|---|---|
| Einsatz Edukt | destilliert | destilliert |

| **GC-Analyse Edukt (%)** | | |
|---|---|---|
| Vorlauf/C4-C7-Kohlenwasserstoffe | 0,3 | 0,4 |
| andere C8-Olefine | 5,9 | 7,7 |
| 2-Ethyl-1-hexen | 9,3 | 9,2 |
| cis-3-Methyl-3-hepten | 15,2 | 15,0 |
| trans-3-Methyl-3-hepten | 27,4 | 27,1 |
| cis-3-Methyl-2-hepten | 16,1 | 15,6 |
| trans-3-Methyl-2-hepten | 25,2 | 24,7 |
| n-Octene | 0,5 | 0,2 |
| Nachlauf | 0,1 | 0,1 |

| **Versuchsbedingungen** | | |
|---|---|---|
| Rh-Konzentration [ppm], bezogen auf Octeneinsatz | 20 | 10 |
| Druck [MPa] | 19 | 27 |
| Temperatur [°C] | 140 | 140 |
| Reaktionszeit [h] | 2 | 2 |

| **GC-Analyse Produkt (%)** | | |
|---|---|---|
| Vorlauf | 0,1 | 0,1 |
| C8-Kohlenwasserstoffe | 2,5 | 1,1 |
| Zwischenlauf | 0,3 | 0,1 |
| iso-Nonanale | 90,8 | 94,7 |
| n-Nonanal | 2,0 | 1,4 |
| Nachlauf | 4,3 | 2,6 |

Die unter Verwendung von Triphenylphosphin als Komplexligand durchgeführten Hydroformylierungsversuche mit dem über die Dehydratisierung von 2-Ethylhexanol erhaltenen Octen sind in der nachfolgenden Tabelle 2 zusammengestellt. Es kam undestillierte Ware zum Einsatz. Die Einsatzoctene sowie die erhaltenen Reaktionsprodukte wurden gaschromatographisch analysiert (Angaben in FI.-%, gemäß DIN 51405).

**Tabelle 2: Hydroformylierung von Octenen, erhalten durch die 2-Ethylhexanoldehydratisierung, Zusatz von Triphenylphosphin**

| **Beispiel** | **IIc** | **IId** | **IIe** | **IIf** |
|---|---|---|---|---|
| Einsatz Edukt | undestilliert, roh | undestilliert, roh | undestilliert, roh | undestilliert, roh |

| **GC-Analyse Edukt (%)** | | | | |
|---|---|---|---|---|
| C4-C7 Kohlenwasserstoffe | 0,3 | 0,3 | 0,3 | 0,4 |
| Andere C8-Olefine | 19,1 | 19,1 | 19,1 | 11,6 |
| 2-Ethyl-1-hexen | 7,9 | 7,9 | 7,9 | 8,6 |
| 3-Methyl-3-hepten | 36,5 | 36,5 | 36,5 | 40,0 |
| 3-Methyl-2-hepten | 36,2 | 36,2 | 36,2 | 39,3 |
| Nachlauf | <0,01 | <0,01 | <0,01 | 0,1 |

| **Versuchsbedingungen** | | | | |
|---|---|---|---|---|
| Rh-Konzentration [ppm], bezogen auf Octeneinsatz | 10 | 10 | 10 | 10 |
| Äquivalente TPP | 3 | 50 | 100 | 3 |
| Druck [MPa] | 18 | 27 | 18 | 14 |
| Temperatur [°C] | 140 | 140 | 140 | 160 |
| Reaktionszeit [h] | 1 | 2 | 1 | 2 |

| **GC-Analyse Produkt (%)** | | | | |
|---|---|---|---|---|
| Vorlauf | 0,1 | 0,1 | 0,1 | 0,1 |
| C8 Kohlenwasserstoffe | 52,2 | 70,9 | 81,7 | 14,1 |
| Zwischenlauf | 0,8 | 0,1 | 0,1 | 1,9 |
| Iso-Nonanale | 45,7 | 28,3 | 17,6 | 76,1 |
| n-Nonanal | 0,5 | 0,1 | 0,1 | 0,5 |
| Nachlauf | 0,7 | 0,4 | 0,4 | 7,3 |

Aus dem nach Beispiel IIa erhaltenen Isononanal wurden zunächst Leichtsieder und unumgesetztes Olefin als Kopfprodukt an einer 24 Bödenkolonne bei 200 hPa, einer Sumpftemperatur von 120°C und einem Rücklaufverhältnis von 2:1 abgetrennt. Nach Leichtsiederabtrennung wurde die Sumpftemperatur auf 140-150°C angezogen und das Isononanal über Kopf abgezogen (Siedepunkt bei 100 hPa: 110-114°C), während Hochsieder im Destillationssumpf verblieben.

Das erhaltene Isononanal wies folgende gaschromatographisch ermittelte Zusammensetzung auf und wurde für die nachfolgende Hydrierung eingesetzt.

**Tabelle 3: Gaschromatographische Analyse (FI.-%, gemäß DIN 51405) des Isononanals ausgehend von 2-Ethylhexanol**

| | |
|---|---|
| Vorlauf/C8-Kohlenwasserstoffe | 0,2 |
| Zwischenlauf | 0,4 |
| 2-Ethyl-4-methylhexanal | 10,8 |
| 2-Propyl-3-methylpentanal | 3,6 |
| 2,5-Dimethylheptanal | 21,9 |
| 2,3-Dimethylheptanal (Isomer) | 4,8 |
| 2,3-Dimethylheptanal (Isomer) + 2-Ethylheptanal | 8,4 |
| 2-Methyloctanal | 1,7 |
| 3-Ethylheptanal | 10,4 |
| 4-Methyloctanal | 20,6 |
| 4,5-Dimethylheptanal | 0,6 |
| 6-Methyloctanal | 11,0 |
| andere i-Nonanale | 1,8 |
| n-Nonanal | 0,9 |
| Nachlauf | 2,9 |

### III. Hydrierung des nach Schritt II. erhaltenen Isononanals zu Isononanol

Das nach Schritt IIa erhaltene und aufgereinigte Isononanal wurde zusammen mit dem kommerziell verfügbaren Nickelkatalysator Ni 55/5 von Johnson Matthey, der in einer Menge von 6 Gew.-%, bezogen auf den Reaktionsansatz, eingesetzt wurde, in einem Autoklaven vorgelegt und bei einem Wasserstoffdruck von 10 MPa und bei einer Temperatur von 100-130°C über zwei Stunden hydriert.

Das nach Filtration des Katalysators erhaltene Rohprodukt wies folgende gaschromatographisch ermittelte Zusammensetzung auf (FI.-%, gemäß DIN 51405):

| | |
|---|---|
| Vorlauf | 1,3 |
| Zwischenlauf | 5,0 |
| Isononanol | 87,7 |
| n-Nonanal | 0,6 |
| Nachlauf | 5,4 |

### IV. Ammonolyse des nach Schritt III. erhaltenen Isononanols zu Isononylamin

Das nach Schritt III. erhaltene Isononanol wurde zusammen mit dem kommerziell verfügbaren Nickelkatalysator Ni 52/35 von Johnson Matthey, der in einer Menge von 10 Gew.-%, bezogen auf den Reaktionsansatz eingesetzt wurde, in einem Autoklaven vorgelegt. Anschließend wurde ein Wasserstoffdruck von 1,5 MPa eingestellt und Ammoniak in einem Molverhältnis von 8 zu 1, bezogen auf Isononanol, zudosiert.

Das Reaktionsgemisch wurde auf 250°C aufgeheizt und durch Wasserstoffdosierung auf einen Druck von 29 MPa gebracht. Nach acht Stunden Reaktionszeit wurde das Reaktionsgemisch entspannt und von Katalysator abfiltriert.

Das erhaltene Rohprodukt wies folgende gaschromatographisch ermittelte Zusammensetzung auf (FI.-%, gemäß DIN 51405):

| | |
|---|---|
| Vorlauf | 7,6 |
| Isononanol | 13,3 |
| Isononylamin | 30,3 |
| Zwischenlauf | 8,5 |
| Di-isononylamin* | 35,1 |
| Zwischenlauf | 1,6 |
| Tri-isononylamin | 3,5 |
| Nachlauf/Höhersieder | 0,1 |

| | |
|---|---|
| *einschließlich Schiffsche Base enthaltend den Isononylrest | |

### V. Aminierung des nach Schritt II. erhaltenen Isononanals zu Isononylamin

Das nach Schritt IIa erhaltene und aufgereinigte Isononanal wurde zusammen mit dem kommerziell verfügbaren Nickelkatalysator Ni 52/35 von Johnson Matthey, der in einer Menge von 5 Gew.-%, bezogen auf den Reaktionsansatz, eingesetzt wurde, in einem Autoklaven vorgelegt und bei einem Wasserstoffdruck von 10,2 MPa und bei einer Temperatur von 120°C in Gegenwart von 10 mol Ammoniak pro mol Isononanal über einen Zeitraum von vier Stunden aminierend hydriert.

Nach Entspannung des Reaktionsansatzes filtrierte man vom Nickelkatalysator und gab das Reaktionsgemisch in einen Phasentrenner, in dem sich das gebildete Reaktionswasser von der organischen Phase separierte. Das organische Rohprodukt wies folgende gaschromatographisch ermittelte Zusammensetzung auf (FI.-%, gemäß DIN 51405):

| | |
|---|---|
| Vorlauf | 1,2 |
| Isononanol | 28,3 |
| Isononylamin | 59,8 |
| Zwischenlauf | 0,6 |
| Di-isononylamin* | 5,6 |
| Zwischenlauf | 0,4 |
| Tri-isononylamin | 2,5 |
| Nachlauf/Höhersieder | 1,6 |

| | |
|---|---|
| *einschließlich Schiffsche Base enthaltend den Isononylrest | |

### VI. Herstellung von Di-isononvlamin über die entsprechende Schiffsche Base durch Umsetzung des nach Schritt V. erhaltenen Isononylamins mit dem nach Schritt IIa erhaltenen und aufgereinigten Isononanal

Zur Herstellung der Schiffschen Base wurde das gemäß Schritt V. erhaltene Isononylamin in einem Reaktionsgefäß vorgelegt und mit dem gemäß Schritt IIa erhaltenen und aufgereinigten Isononanal bis zu einem Molverhältnis von 1 mol Isononylamin zu 1,1 mol Isononanal tropfenweise versetzt. Nach einer Reaktionszeit von dreieinhalb Stunden bei Raumtemperatur gab man das Reaktionsgemisch in einen Phasentrenner, in dem sich das gebildete Reaktionswasser von der organischen Phase separierte. Die organische Phase enthaltend die Schiffsche Base wurde zusammen mit dem kommerziell verfügbaren Nickelkatalysator Ni 52/35 von Johnson Matthey, der in einer Menge von 5 Gew.-%, bezogen auf den Reaktionsansatz, eingesetzt wurde, in einem Autoklaven vorgelegt und bei einem Wasserstoffdruck von 10 MPa und bei einer Temperatur von 120°C über einen Zeitraum von sechs Stunden hydriert. Das nach Filtration des Katalysators erhaltene Rohprodukt wies folgende gaschromatographisch ermittelte Zusammensetzung auf (FI.-%, gemäß DIN 51405):

| | |
|---|---|
| Vorlauf | 9,7 |
| Isononanol | 3,9 |
| Isononylamin | 4,5 |
| Zwischenlauf | 4,8 |
| Di-isononylamin* | 62,6 |
| Zwischenlauf | 0,4 |
| Tri-isononylamin | 14,0 |
| Nachlauf/Höhersieder | 0,1 |

| | |
|---|---|
| *einschließlich Schiffsche Base enthaltend den Isononylrest | |

### VII. Herstellung von Tri-isononvlamin durch Ammonolvse des nach Schritt III erhaltenen Isononanols

Das nach Schritt III. erhaltene Isononanol wurde zusammen mit dem konventionell verfügbaren Nickelkatalysator Ni 55/5 von Johnson Matthey, der in einer Menge von 5 Gew.-%, bezogen auf den Reaktionsansatz, eingesetzt wurde, in einem Rührbehälter vorgelegt. Anschließend wurde unter Normaldruck Wasserstoff in einer Menge von 5 Normliter pro Stunde durch die Reaktionslösung geleitet und parallel dazu Ammoniak in einer Menge von 0,9 mol Ammoniak pro mol Isononanol über einen Zeitraum von drei Stunden zudosiert. Das entstehende Reaktionswasser wurde azeotrop ausgekreist. Nach drei Stunden beendete man die Reaktion und filtrierte den Katalysator ab. Das erhaltene Rohprodukt wies folgende gaschromatographisch ermittelte Zusammensetzung auf (FI.-%, gemäß DIN 51405):

***einschließlich Schiffsche Base enthaltend den Isononylrest**

| | |
|---|---|
| Vorlauf | 1,5 |
| Isononanol | 6,8 |
| Isononylamin | 1,4 |
| Di-isononylamin* | 1,1 |
| Zwischenlauf | 0,1 |
| Tri-isononylamin | 89,0 |
| Nachlauf/Höhersieder | 0,1 |

## Patentansprüche

1. Verfahren zur Herstellung von Isononylaminen ausgehend von 2-Ethylhexanol, **dadurch gekennzeichnet, dass** man
(a) 2-Ethylhexanol in Gegenwart eines Katalysators zu Octen dehydratisiert;
(b) das nach Schritt a) erhaltene Octen in Gegenwart einer Übergangsmetallverbindung der Gruppe VIII des Periodensystems der Elemente mit Kohlenmonoxid und Wasserstoff zu Isononanal umsetzt; und
(c) das nach Schritt b) erhaltene Isononanal in Isononylamine überführt.

2. Verfahren gemäß Anspruch 1, **dadurch gekennzeichnet, dass** man in Schritt a) als Katalysator Aluminiumoxid, Nickel niedergeschlagen auf Aluminiumoxid, oder Phosphorsäure niedergeschlagen auf Siliziumdioxid oder Aluminiumoxid verwendet.

3. Verfahren gemäß Anspruch 1 oder 2, **dadurch gekennzeichnet, dass** man in Schritt a) 2-Ethylhexanol in der Gasphase dehydratisiert.

4. Verfahren gemäß einem oder mehreren der Ansprüche 1 bis 3, **dadurch gekennzeichnet, dass** man in Schritt b) als Übergangsmetallverbindung der Gruppe VIII des Periodensystems der Elemente eine Kobalt- oder Rhodiumverbindung verwendet.

5. Verfahren gemäß einem oder mehreren der Ansprüche 1 bis 4, **dadurch gekennzeichnet, dass** die Umsetzung gemäß Schritt b) in Abwesenheit von komplexbildenden Organoelementverbindungen durchgeführt wird.

6. Verfahren gemäß einem oder mehreren der Ansprüche 1 bis 5, **dadurch gekennzeichnet, dass** man das nach Schritt b) erhaltene Isononanal destilliert.

7. Verfahren gemäß einem oder mehreren der Ansprüche 1 bis 6, **dadurch gekennzeichnet, dass** man in Schritt c) das nach Schritt b) erhaltene Isononanal mit Ammoniak, einem primären oder sekundären Amin und Wasserstoff in Gegenwart eines Aminierungskatalysators zu Isononylaminen umsetzt.

8. Verfahren gemäß einem oder mehreren der Ansprüche 1 bis 6, **dadurch gekennzeichnet, dass** man in Schritt c) das nach Schritt b) erhaltene Isononanal mit Wasserstoff in Gegenwart eines Hydrierkatalysators zu Isononanol hydriert und anschließend mit Ammoniak, einem primären oder sekundären Amin und Wasserstoff in Gegenwart eines Aminierungskatalysators zu Isononylaminen umsetzt.

9. Verfahren gemäß einem oder mehreren der Ansprüche 1 bis 8, **dadurch gekennzeichnet, dass** man als Aminierungskatalysator einen Nickel- oder Kobaltkatalysator verwendet.

## Claims

1. Process for preparing isononylamines starting out from 2-ethylhexanol, **characterized in that**
(a) 2-ethylhexanol is dehydrated in the presence of a catalyst to form octene;
(b) the octene obtained in step a) is reacted with carbon monoxide and hydrogen in the presence of a transition metal compound of group VIII of the Periodic Table of the Elements to form isononanal; and
(c) the isononanal obtained in step b) is converted into isononylamines.

2. Process according to Claim 1, **characterized in that** aluminium oxide, nickel deposited on aluminium oxide, or phosphoric acid deposited on silicon dioxide or aluminium oxide is used as catalyst in step a).

3. Process according to Claim 1 or 2, **characterized in that** 2-ethylhexanol is dehydrated in the gas phase in step a).

4. Process according to one or more of Claims 1 to 3, **characterized in that** a cobalt or rhodium compound is used as transition metal compound of group VIII of the Periodic Table of the Elements in step b).

5. Process according to one or more of Claims 1 to 4, **characterized in that** the reaction in step b) is carried out in the absence of complexing organoelement compounds.

6. Process according to one or more of Claims 1 to 5, **characterized in that** the isononanal obtained in step b) is distilled.

7. Process according to one or more of Claims 1 to 6, **characterized in that** the isononanal obtained in step b) is, in step c), reacted with ammonia, a primary or secondary amine and hydrogen in the presence of an amination catalyst to form isononylamines.

8. Process according to one or more of Claims 1 to 6, **characterized in that** the isononanal obtained in step b) is, in step c), hydrogenated by means of hydrogen in the presence of a hydrogenation catalyst to form isononanol and subsequently reacted with ammonia, a primary or secondary amine and hydrogen in the presence of an amination catalyst to form isononylamines.

9. Process according to one or more of Claims 1 to 8, **characterized in that** a nickel or cobalt catalyst is used as amination catalyst.

## Revendications

1. Procédé de fabrication d'isononylamines à partir de 2-éthylhexanol, **caractérisé en ce que**
(a) du 2-éthylhexanol est déshydraté en présence d'un catalyseur en octène ;
(b) l'octène obtenu à l'étape a) est mis en réaction en présence d'un composé de métal de transition du groupe VIII du tableau périodique des éléments avec du monoxyde de carbone et de l'hydrogène pour former de l'isononanal ; et
(c) l'isononanal obtenu à l'étape b) est transformé en isononylamines.

2. Procédé selon la revendication 1, **caractérisé en ce que** de l'oxyde d'aluminium, du nickel déposé sur de l'oxyde d'aluminium ou de l'acide phosphorique déposé sur du dioxyde de silicium ou de l'oxyde d'aluminium est utilisé en tant que catalyseur à l'étape a).

3. Procédé selon la revendication 1 ou 2, **caractérisé en ce que** le 2-éthylhexanol est déshydraté dans la phase gazeuse à l'étape a).

4. Procédé selon une ou plusieurs des revendications 1 à 3, **caractérisé en ce qu'**un composé de cobalt ou de rhodium est utilisé à l'étape b) en tant que composé de métal de transition du groupe VIII du tableau périodique des éléments.

5. Procédé selon une ou plusieurs des revendications 1 à 4, **caractérisé en ce que** la réaction selon l'étape b) est réalisée en l'absence de composés d'organoéléments complexants.

6. Procédé selon une ou plusieurs des revendications 1 à 5, **caractérisé en ce que** l'isononanal obtenu à l'étape b) est distillé.

7. Procédé selon une ou plusieurs des revendications 1 à 6, **caractérisé en ce qu'**à l'étape c), l'isononanal obtenu à l'étape b) est mis en réaction avec de l'ammoniac, une amine primaire ou secondaire et de l'hydrogène en présence d'un catalyseur d'amination pour former des isononylamines.

8. Procédé selon une ou plusieurs des revendications 1 à 6, **caractérisé en ce qu'**à l'étape c), l'isononanal obtenu à l'étape b) est hydrogéné avec de l'hydrogène en présence d'un catalyseur d'hydrogénation pour former de l'isononanol, puis mis en réaction avec de l'ammoniac, une amine primaire ou secondaire et de l'hydrogène en présence d'un catalyseur d'amination pour former des isononylamines.

9. Procédé selon une ou plusieurs des revendications 1 à 8, **caractérisé en ce qu'**un catalyseur de nickel ou de cobalt est utilisé en tant que catalyseur d'amination.
